# EUROPEAN PATENT SPECIFICATION

(11) **EP 4 373 549 B1**
(45) Date of publication and mention of the grant of the patent: **07.10.2026**
(21) Application number: 22754310.5
(22) Date of filing: 05.07.2022
(51) Int. Cl.: A61M 5/20, A61M 5/32, A61M 5/24, A61M 5/315

(54) **A SUBASSEMBLY OF A MEDICAMENT DELIVERY DEVICE**
UNTERANORDNUNG EINER MEDIKAMENTENABGABEVORRICHTUNG
SOUS-ENSEMBLE D'UN DISPOSITIF D'ADMINISTRATION DE MÉDICAMENT

(30) Priority: 22.07.2021 US 202163224440 P; 26.08.2021 EP 21193368
(43) Date of publication of application: 29.05.2024
(73) Proprietor: SHL Medical AG, 6302 Zug (CH)
(72) Inventor: HUANG, Chun-Hsien, Taoyuan City, 338 (TW); BAKHTIYARI, Ali, Taoyuan City, 338 (TW)
(86) International application number: PCT/EP2022/068539
(87) International publication number: WO 2023/001540

(56) References cited:
- CA-A1- 3 123 210
- US-A1- 2003 114 789
- US-A1- 2016 106 920
- US-A1- 2017 368 259
- US-A1- 2019 298 924
- US-B2- 9 833 579

## Description

### TECHNICAL FIELD

The present disclosure generally relates to subassembly of a medicament delivery device for expelling medicament from a medicament container.

### BACKGROUND

Autoinjectors or pen-injectors require a source of physical power for pushing a plunger to expel the medicament. The source of power may be provided by human force, springs, electrical motors, or a combination thereof.

Spring are often used but are limited in their performance due to the limited non-linear force they provide. Further, springs for autoinjectors are typically associated with complex manufacturing procedures.

Attempts have been made in using electrical motors since they can be configured to more accurately control the force applied to the plunger. However, a high output force from an electrical motor, e.g. step motor, requires high electricity consumption, and may therefore require a user to charge or change a battery every time before use. Relevant prior art can be found under US 2016/00106920 A1 on a spring driven auto injector with a front needle shield trigger, US 9,833,579 B2 on a mechanical cartridge based injection device, US 2019/00298924 A1 on a delayed delivery system with a needle sleeve triggering an electromechanical actuator, US 2017/00368259 A1, CA 3,123,210 A1 and US 2003/00114789 A1.

### SUMMARY

It would be desirable to provide a means for controlling an activation of an electrical motor in a reliable and at the same time cost-efficient way. The invention is defined in the appended claims.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the components thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", "circumferentially" refer to a circumference or a circumferential direction relative to an axis, typically a central axis extending in the direction of the longest extension of the device and/or component. Similarly, "radial" or "radially" refer to a direction extending radially relative to the axis, and "rotation", "rotational" and "rotationally" refer to rotation relative to the axis.

According to the invention, there is hence provided a subassembly of a medicament delivery device for expelling medicament from a medicament container, the subassembly comprising: a housing having a proximal end and a distal end; an electrical engine configured to, upon activation, cause expulsion of the medicament out from the medicament container; in preferred embodiments, the electrical engine comprises liquid substance and is configured to generate propellant gas from the liquid substance upon being activated; according to the invention, the subassembly further comprises an electronic triggering device configured to activate the electrical engine, the electrical engine and the electronic triggering device being accommodated in the housing; the electronic triggering device is configured to sense a movable trigger comprising a sensing area in response to sensing the sensing area, the electronic triggering device is configured to provide a triggering signal to activate the electrical engine.

The electrical engine is preferably a miniature machine, e.g. a MEMS (micro electrical-mechanical system) machine, that comprises both electrical and mechanical components. Various such electrical engines are known *per se.*

The electrical engine is preferably configured to generate the propellant gas by electrochemistry way, e.g. electrolysis. Alternative, the electrical engine is configured to generate the propellant gas by heating the liquid substance and generate vapor.

The subassembly is therefore able to be more compact and can generate a high force. The subassembly is thus suitable to be a powerpack of the medicament delivery device.

According to one embodiment, the propellant gas is configured to flow into the medicament container and propel a plunger in the proximal direction of the medicament container.

According to one embodiment, the propellant gas is configured to propel a plunger of the medicament container in the proximal direction of the medicament container.

According to one embodiment, the subassembly comprises a plunger for expelling a contained medicament in the medicament container, so that the propellant gas is configured to flow into a tubular body of the plunger when the electrical engine is trigger.

According to one embodiment, subassembly comprises a medicament container holder for holding the medicament container; the medicament container holder is gas-tightly sealed to the electrical engine.

According to one embodiment, the subassembly comprises a resilient ring positioned between the medicament container and holder and the electrical engine.

According to one embodiment, the medicament container holder is connected to the electrical engine through a screw thread engagement, a bayonet engagement, or a snap-fit engagement.

According to one embodiment, the propellant gas is configured to flow into a sleeve connected to either a tubular plunger or a solid core plunger rod.

According to the invention, , the electronic triggering device comprises an electronic non-contact sensor.

According to one embodiment, the electronic non-contact sensor comprising an emitter configured to transmit a signal and a detector configured to detect a reflected signal from the sensing area, whereby the triggering signal is provided in response to detecting the reflected signal.

According to one embodiment, the electronic non-contact sensor is an optical sensor or an acoustic sensor.

According to one embodiment, the electronic non-contact sensor being a magnetic field sensor configured to detect a magnetic element in the sensing area, the electronic triggering device is configured to provide a triggering signal when the detected magnetic field strength is above a detection threshold.

According to one embodiment, the electronic non-contact sensor is configured to sense the movable trigger by detecting a variation in detection signal caused by a motion of the sensing area.

According to one embodiment, the electronic triggering device comprises a mechanical switch.

According to the invention, , the subassembly comprising a cover structure configured to surround a medicament delivery member at the proximal end of the housing, the cover structure is movable in a longitudinal direction of the housing between a retracted position in which the medicament delivery member is exposed at the proximal end of the cover structure, and a extended position with the medicament delivery member covered by the cover structure.

According to the invention, , the cover structure comprises the sensing area, the electronic triggering device is configured to sense that the cover structure is in the retracted position, in response to sensing that the cover structure is in the retracted position, the electronic triggering device is configured to provide a triggering signal to activate the electrical engine. According to one embodiment, the cover structure is arranged such that, when the cover structure is moved towards the retracted position, a distance between the sensing area and the electronic non-contact sensor is reduced, thereby causing a variation in detection signal.

According to one embodiment, the electronic non-contact sensor is configured to sense the movable trigger by detecting an appearance of the cover structure.

Another aspect of the invention provides a subassembly of a medicament delivery device for expelling medicament from a medicament container, the subassembly comprising: a housing having a proximal end and a distal end; an electrical engine configured to, upon activation, cause expulsion of the medicament out from the medicament container, an electronic triggering device configured to activate the electrical engine, the electrical engine and the electronic triggering device being accommodated in the housing; the electronic triggering device comprises an electronic non-contact sensor configured to sense a movable trigger comprising a sensing area wherein, in response to sensing the sensing area, the electronic triggering device is configured to provide a triggering signal to activate the electrical engine. The electronic non-contact sensor provides for a reliable sensing means for detecting the sensing area and thereby triggering the electrical engine to activate. Such electronic non-contact sensor provides for a sensor that do not have to include moving mechanical parts in contact with the movable trigger, thereby eliminating potential sources of mechanical failure. Further, using an electronic based non-contact sensor advantageously provides for accurate and reproducible timing of the activation of the engine that do not have to rely on mechanical trigger links to the movable trigger.

According to one embodiment, the electronic non-contact sensor may comprise an emitter configured to transmit a signal and a detector configured to detect a reflected signal from the sensing area, whereby the triggering signal is provided in response to detecting the reflected signal.

According to one embodiment, the electronic non-contact sensor may be an optical sensor, the emitter being configured to emit an optical signal and the receiver being configured to detect a reflected optical signal. Optical sensors provide for an accurate and cost-efficient electronic non-contact sensor. Further, optical sensors are low power consumers and are relatively small and thus able to fit into the subassembly.

For example, the optical sensor may be an infrared sensor, thus emitting an infrared optical signal and being adapted to detect an infrared optical signal.

In one embodiment, the sensing area may comprise an optically reflective surface. This advantageously improves the detection accuracy of an optical sensor relying on detecting a reflected signal.

According to one embodiment, the electronic non-contact sensor may be an ultrasound sensor, the emitter being configured to emit an ultrasound signal and the receiver being configured to detect a reflected ultrasound signal. Using ultrasound sensors is a further alternative accurate and cost-efficient electronic non-contact sensor, that can be fitted into the subassembly. The detected ultrasound signal has been reflected from the sensing area of the movable trigger.

According to one embodiment, the electronic non-contact sensor may be a magnetic field sensor configured to detect a magnetic element in the sensing area, wherein the electronic triggering device is configured to provide a triggering signal when the detected magnetic field strength is above a detection threshold. Accordingly, the magnetic element is spatially transferred by the movable trigger, and, once sufficiently close to the magnetic field sensor, is detected by the magnetic field sensor according to some detection threshold. The magnetic element comprises a magnetic material.

In one example, the magnetic field sensor may a Hall effect sensor.

According to one embodiment, the electronic non-contact sensor may be configured to sense the movable trigger by detecting an appearance of the sensing area. In one example, the electronic non-contact sensor may a camera device adapted to detect the appearance of the sensing area by detecting specific feature in the sensing area. The features may be detected in an image captured by the camera.

According to one embodiment, the electronic non-contact sensor may be configured to sense the movable trigger by detecting a variation in detection signal caused by a motion of the sensing area. Accordingly, as the sensing area moves, the detection signal varies, whereby a variation exceeding a threshold may be an indication that the engine should be activated.

According to one embodiment, the electrical engine may be configured to cause a plunger to move in the medicament container by the force from the electrical engine when the electrical engine is activated.

According to one embodiment, once activated, the electrical engine may be configured to remain activated only for a predetermined time duration. Hereby, reduced power consumption is achieved.

According to one embodiment, the electronic triggering device may be configured to deactivate the electrical engine when the electronic non-contact sensor no longer detects the proximity of the sensing area. Deactivation of the electrical engine provides for reduced power consumption.

According to one embodiment, the electronic triggering device may comprise processing circuitry configured to receive a sensing signal from the electronic non-contact sensor indicative of the sensing area being sensed, and in response to provide an activation signal to the electrical engine.

According to one embodiment, the subassembly may comprise power source configured to power the electrical engine and the electronic triggering device.

According to one embodiment, the subassembly may comprise an inner frame arranged in the housing at the distal end, the inner frame being arranged to support the electrical engine, a power source, and the electronic triggering device. Hereby, a modular subassembly is provided.

According to one embodiment, the housing may comprise a first housing part and a second housing part attachable to each other, wherein the first housing part is a distal housing part accommodating the inner frame, and the second housing part is a proximate housing part configured to accommodate at least part of a cover structure for a medicament delivery member, and the medicament container.

According to the invention, the subassembly may comprise a cover structure configured to surround a medicament delivery member at the proximal end of the housing, the cover structure is movable in a longitudinal direction of the housing between a retracted position in which the medicament delivery member is exposed at the proximal end of the cover structure, and an extended position with the medicament delivery member covered by the cover structure. Thus, the cover structure is adapted to cover the medicament delivery member, such as a needle, so that a user is protected from non-intended needlesticks. In the extended position, the delivery member may for example be moved fully into the cover structure.

According to the invention, the cover structure may comprise the sensing area, wherein the electronic triggering device is configured to sense that the cover structure is in the retracted position, wherein, in response to sensing that the cover structure is in the retracted position, the electronic triggering device is configured to provide a triggering signal to activate the electrical engine. Thus, the movable trigger is part of the cover structure thereby directly linking the motion or position of the cover structure to the activation of the electrical engine.

According to one embodiment, the cover structure may be arranged such that, when the cover structure is moved towards the retracted position, a distance between the sensing area and the electronic non-contact sensor is reduced, thereby causing a variation in detection signal.

According to one embodiment, the electronic non-contact sensor may be configured to sense the movable trigger by detecting an appearance of the cover structure.

According to one embodiment, the electronic non-contact sensor may be arranged spaced apart from the sensing area such that the cover structure can slide freely over the electronic non-contact sensor.

According to an example not forming part of the invention, the subassembly may comprise a push button configured to actuate the movable trigger. The push button may be actuated by a user pushing the button.

According to an example not forming part of the invention, the push button is configured to be moved by a user pushing the button in a longitudinal direction of the housing from an initial position to a triggered position.

According to an example not forming part of the invention, the push button comprises the sensing area.

According to an example not forming part of the invention, the electronic triggering device is configured to sense that the push button is in the triggered position, wherein, in response to sensing that the push button is in the triggered position, the electronic triggering device is configured to provide a triggering signal to activate the electrical engine.

According to one embodiment, the subassembly may comprise a rotator being rotatable by the cover structure to a locking position when the cover structure moves from the extended position to the retracted position, whereby, in the locking position, the rotator is configured to lock the cover structure in the extended position after use of the medicament delivery device.

According to one embodiment, the rotator may comprise a first track for receiving a guiding pin of the cover structure, wherein the guiding pin is arranged to move along the first track when the cover structure moves from the extended position to the retracted position to cause the rotation of the rotator, the rotator comprising a second track configured to receive the guiding pin from the first track when the cover structure moves from the retracted position to the extended position, whereby once the guiding pin is received in the second track, the cover structure is locked in the retracted position.

According to one embodiment, the electrical engine may be configured to generate propellant gas upon being activated.

According to one embodiment, the electrical engine may comprise liquid substance and configured to generate propellant gas from the liquid substance upon being activated. The generation of propellant gas may be based on e.g. electrolysis or heating processes of the liquid substance.

According to one embodiment, the propellant gas may be configured to flow into the medicament container and propel a plunger in the proximal direction of the medicament container.

According to one embodiment, the propellant gas may be configured to propel a plunger.

According to one embodiment, the propellant gas may be configured to flow into a tubular body of the plunger.

According to one embodiment, the propellant gas may be configured to flow into a sleeve connected to either a tubular plunger or a solid core plunger rod.

There is according to another aspect of the present disclosure provided a subassembly module for controlling expulsion of medicament from a medicament container, the subassembly module comprising: a frame structure arrangeable in a housing having a proximal end and a distal end; an electrical engine configured to, upon activation, cause expulsion of the medicament out from the medicament container, an electronic triggering device configured to activate the electrical engine; a power source configured to power the electrical engine and the electronic triggering device, the frame being configured to accommodate the electrical engine, the power source, and the electronic triggering device, wherein the electronic triggering device comprises an electronic non-contact sensor configured to sense a movable trigger comprising a sensing area, in response to sensing the sensing area the electronic triggering device is configured to provide a triggering signal to activate the electrical engine.

Hereby an advantageous subassembly module is provided with advantages related to the electronic non-contact sensor as discussed above.

According to one embodiment, the frame may comprise an inner space for accommodating the electrical engine, the power source, and the electronic triggering device.

According to one embodiment, the medicament delivery device that can comprise the subassembly of the invention can be an injector or an inhaler.

According to one embodiment, the injector can be a pen-type auto-injector, an on-body injector, or an infusion pump.

According to one embodiment, the medicament container can be a syringe or a cartridge.

According to one embodiment, the electrical engine comprises an opening cover is covered by a movable lid.

Generally, all terms used in the claims are to be interpreted according to their ordinary meaning in the technical field, unless explicitly defined otherwise herein. All references to "a/an/the member, apparatus, component, means, etc." are to be interpreted openly as referring to at least one instance of the member, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 is a perspective view of an example of a medicament delivery device;
Fig. 2 is an exploded view of the medicament delivery device in Fig. 1;
Figs 3A-3D show longitudinal sections of the medicament delivery device during various stages of operation;
Figs 4A-4B show longitudinal sections of the medicament delivery device during various stages of operation when triggering the actuation of the electrical engine using a non-contact sensor; and
Figs 5A-5B show longitudinal sections of the medicament delivery device during various stages of operation when triggering the actuation of the electrical engine using a non-contact sensor.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like members throughout the description.

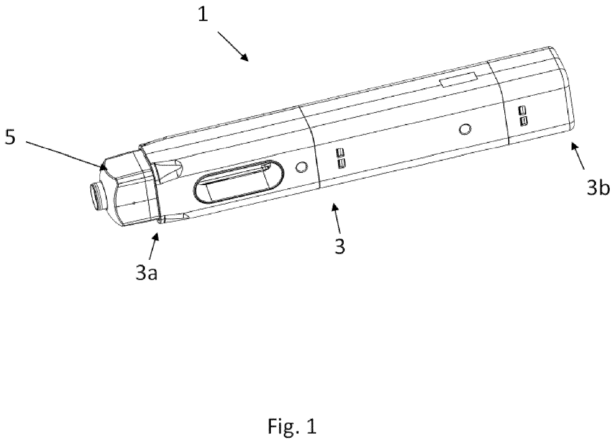

Fig. 1 shows an example of a medicament delivery device 1 configured to expel medicament from a medicament container.

The medicament delivery device 1 comprises a housing 3. The housing 3 has a proximal end 3a and a distal end 3b.

The medicament delivery device 1 comprises a cover structure 5. The cover structure 5 is arranged in the housing 3 and extends proximally from the proximal end 3a.

The cover structure 5 is configured to be moved linearly relative to the housing 3 from a first position, being an extended position shown in Fig. 1 to a second position, being a retracted position in which the cover structure 5 is received further in the housing 3.

The cover structure 5 is biased in the proximal direction towards the first position.

As will be discussed further, a subassembly of the medicament delivery device 1 comprises an electronic triggering device which is configured to provide a triggering signal to activate an electrical engine to discharge medicament once a movable trigger is detected.

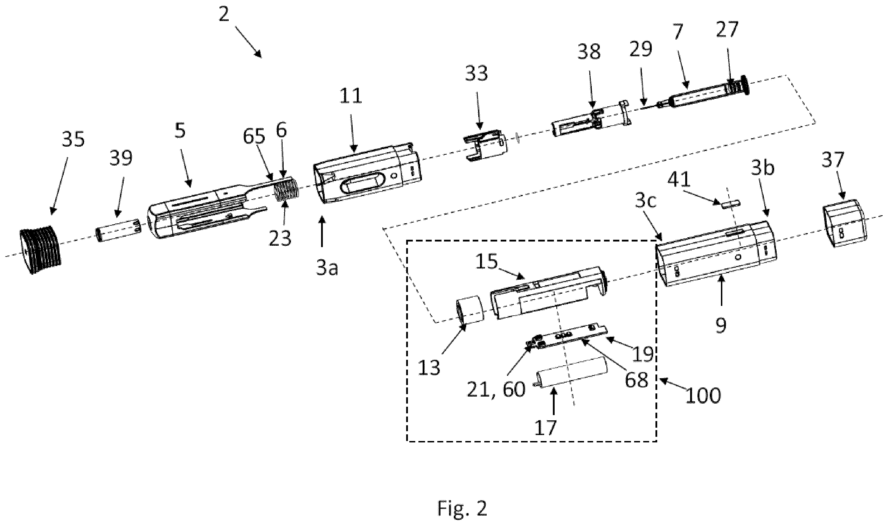

Fig. 2 shows an exploded view of the medicament delivery device 1. A medicament container 7 with which the medicament delivery device 1 can be loaded is also depicted.

A subassembly 2 of the medicament delivery device 1 comprises the housing 3, here being provided as a first housing part 9 and a second housing part 11 attachable to each other.

The subassembly 2 comprises an electrical engine 13 arranged in an inner frame 15. The inner frame 15 is arranged to additionally support a power source 17, and an electronic triggering device 19. The power source, such as a battery, is configured to power the electrical engine 13 and the electronic triggering device 19.

The first housing part 9 is a distal housing part configured to accommodate the inner frame 15. The electrical engine 13 and the electronic triggering device 19 being accommodated in the first housing part 9.

The second housing part 11 is a proximate housing part configured to accommodate at least part of the cover structure 5 for a medicament delivery member such as a needle, and the medicament container 7.

As will be discussed further, the electrical engine 13 is configured to, upon activation, cause expulsion of the medicament out from the medicament container 7. Further, the electronic triggering device 19 is configured to activate the electrical engine 13. For this, the electronic triggering device 19 comprises an electronic non-contact sensor 21 configured to sense a movable trigger 6 comprising a sensing area. In the depicted embodiment, the movable trigger is exemplified as an extension 65 or arm of a cover structure 5. In response to sensing the sensing area, the electronic triggering device 19 is configured to provide a triggering signal to activate the electrical engine. In this example, the extension 65 is a distal portion of the cover structure 5, thus extending in a distal direction subassembly 2. In a preferred example, the extension 65 is a tapered or narrowed part of the cover structure 5 that is adapted to reach to and beyond the location of the electronic non-contact sensor when the cover structure 5 is in the retracted position. In the preferred example, the transversal width of the extension is less than that of the proximal part of the cover structure 5.

The electrical engine 13 being configured to cause a plunger to move in the medicament container 7 by the force from the electrical engine when the electrical engine is activated. The plunger may be part of the electrical engine 13 configured to push a rubber seal 27 of the medicament container 7 into the medicament container 7. In other possible examples, the plunger may be part of the medicament container 7, whereby the plunger is connectable to the electrical engine.

Various implementations for the expulsion of the medicament out from the medicament container 7 are conceivable. In one example, the electrical engine 13 is configured to generate propellant gas upon being activated. In this implementation, the propellant gas may flow directly into the medicament container 7 if the plunger is part of the medicament container 7.

In another example, the electrical engine 13 comprises liquid substance and is configured to generate propellant gas from the liquid substance upon being activated. The generation of propellant gas may be based on e.g. electrochemistry, electrolysis or heating processes of the liquid substance.

In yet another example, the propellant gas is configured to flow into the medicament container 7 and propel a plunger in the proximal direction of the medicament container. In yet another example, the propellant gas is configured to propel a plunger. The propellant gas may for example be configured to flow into a tubular body of the plunger. In another example, the propellant gas is configured to flow into a sleeve connected to the plunger. The plunger of the medicament container 7 is gas-tightly connectable to the electrical engine 13. If the plunger is provided as part of the electrical engine, the plunger is gas-tightly connected to the electrical engine 13.

The plunger may be for example either a tubular plunger or a solid core plunger rod, or a rubber seal of the medicament container when the plunger is a part of the medicament container.

In one example that the plunger is a part of the medicament container, the propellant gas is configured to flow into the medicament container and therefore moves the plunger of the medicament container to expel the contained medicament. In a preferred example, the plunger is the rubber seal of the medicament container, the when the electrical engine 13 is activated, the propellant gas is generated from the electrical engine 13 and flow into the medicament container due to the gas-tightly connection between the plunger and the electrical engine 13. In a preferred example, the gas-tightly connection can be arranged between a medicament container holder and the electrical engine 13. In this example, the medicament container holder is configured to hold the medicament container. Optionally, a resilient ring can be provided between the medicament container holder and the electrical engine; preferably the resilient ring is positioned on a distal edge of the medicament container and the electrical engine, so that a better gas-tight seal can be provided. In a preferred example, the medicament container holder can be attached to the electrical engine via e.g. screw thread engagement, snap-fit engagement, or a bayonet engagement, so that the resilient ring can be squeezed tightly between the electrical engine and the medicament container, thus the plunger of the medicament container is gas-tightly connected to the electrical engine. Furthermore, the screw thread engagement or the bayonet engagement between the medicament container holder and the electrical engine, a medicament delivery device with the subassembly of the invention can be a reusable device, namely, an end user can change the medicament container multiple times, for multiple times of use of the medicament delivery device. It should be noted that, when the plunger is a part of the subassembly, the subassembly is also suitable for a medicament delivery device that is a reusable device. In the example that the plunger is a part of the subassembly, the medicament container holder can be a part of the housing, and no gas-tightly connection between the medicament container holder is needed for the subassembly of the invention.

If the medicament delivery device that comprises the subassembly of the invention is a reusable device, the electrical engine is preferably comprises a opening that is covered by a movable lid, so that the end user or the device manufacturer/ maintenance staff/ recycling staff can refill the liquid into the electrical engine, from the opening, for being further use for generating the propellant gas. The movable lid can be connected to the electrical engine through a hinge or can be completely removed from the electrical engine.

In another example, when the plunger is a part of the subassembly, the subassembly optionally comprises a sleeve connected to either a tubular plunger or a solid core plunger rod. The sleeve is movable relative to the plunger, e.g. either the plunger can move actively relative to the sleeve or the sleeve can move actively relative to the plunger. The sleeve is gas-tightly seal with the plunger and the electrical engine, so that the propellant gas can flow into the sleeve and propel the plunger into the medicament container and expel the contained medicament.

With reference to fig. 2, the subassembly 2 according to the present example comprises the cover structure 5 configured to surround a medicament delivery member 29 at the proximal end 3a of the housing 3 comprising the first housing part 9 and the second housing part 11, here the proximal end being part of the second housing part 11. The cover structure 5 is movable in a longitudinal direction of the housing 3between a retracted position and an extended position. In the retracted position the medicament delivery member is exposed at the proximal end 3a of the cover structure. In the extended position the medicament delivery member is covered by the cover structure 5. The cover structure 5 is biased by a spring 23 towards the extended position. In the retracted position, the extension 65 reaches into the first housing part 9.

The subassembly 2 further comprises a cylindrically shaped rotator 33 being rotatable by the cover structure as the cover structure moves in the longitudinal direction of the subassembly 2. The rotator is adapted to prevent the cover structure from retracting again after an injection event using the subassembly 2. The rotator 33 is rotatable to a locking position when the cover structure moves from the extended position to the retracted position. In the locking position, the rotator 33 is configured to lock the cover structure 5 in the extended position after use of the medicament delivery device.

There is further depicted a removable proximal end cap 35 removable by a user prior to injection, and a distal end cap 37 to seal the subassembly 2. In addition, a holder 38 for the medicament container is also depicted and a removable needle shield holder 39 which may be removable as the user removes the end cap 35. The housing 19is provided with an indication window 41 through which a user can monitor the progress status of a present injection.

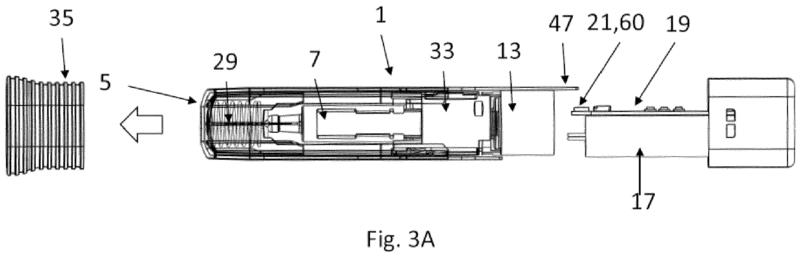

Fig. 3A illustrate a side view of the medicament delivery device 1 in an initial default state being loaded with a medicament container 7 and with the proximal end cap being 35 removed. In figs. 3A-3D the housing is omitted.

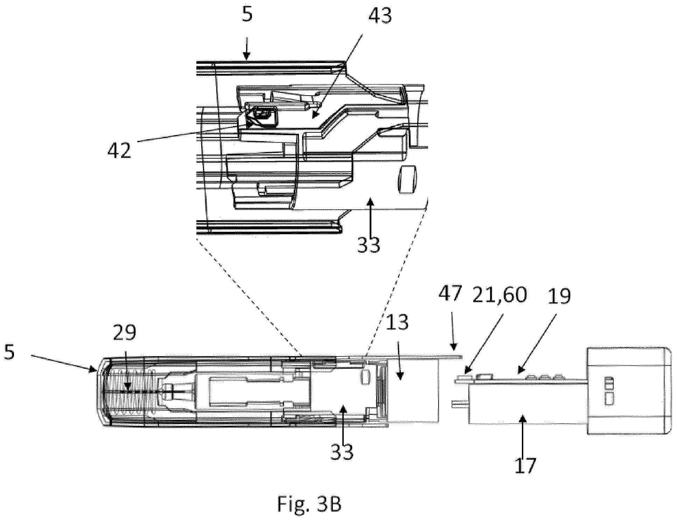

Fig. 3B illustrates a side view the medicament delivery device 1 without the end cap 35 and ready to start an injection event, i.e. placing the cover structure 5 on an injection site on the skin of a user.

In this state, a guiding pin 42 on an inner surface of the cover structure 5 is received in a guiding track 43 of the rotator 33. The track 43 may be provided as a groove in the material on an outer side of the rotator 33 in which the protruding guiding pin 42 may slide. The outer side of the rotator faces the inner surface of the cover structure 5. The medical delivery member, i.e. the needle 29 is still covered by the cover structure 5 being in an extended position.

Further, a sensing area 47 of the cover structure 5, here serving as a movable trigger, is not yet detected by the electronic non-contact sensor 21 in such a way to cause the electronic triggering device 19 to provide a triggering signal. The sensing area 47 is located on a distal end of the cover structure 5. In a preferred example, the sensing area 47 is located on the extension 65 serving as an arm reaching in the distal direction.

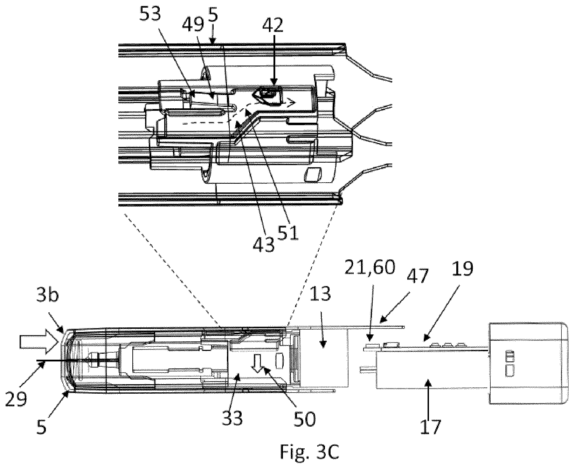

Fig. 3C illustrates a side view the medicament delivery device 1 in an injection state with the cover structure 5 in the retracted position thereby exposing the needle 29 at the proximal end 3a of the medicament delivery device 1. In this state, the electronic non-contact sensor 21 of the electronic triggering device 19 is able to sense the sensing area 47. In response to sensing the sensing area 47, the electronic triggering device 19 is configured to provide a triggering signal to activate the electrical engine 13. Once activated, the electrical engine 13 is configured to cause expulsion of the medicament out from the medicament container 7. The medicament is thereby expelled out through the needle 29 for injection at the injection site.

Further, when the cover structure 5 is moved towards the retracted position, the guiding pin 42moves along the guiding track 43 to beyond, in a distal direction, a locking mechanism 49.. When the guiding pin 42 moves in the guiding track 43, it will make the rotatable rotator 33 rotate as indicated by arrow 50. For this, the guiding track 43 is shaped along a curved path, or along a path that changes direction at least once, here indicated by dashed arrow 51. When the guiding pin 41 moves along the curved path 51, the guiding pin 41 pushes on the rotator 33, in the guiding track 43 to cause the rotator to rotate. The motion of the guiding pin 42 in the guiding track 43 is along the dashed arrow 51.

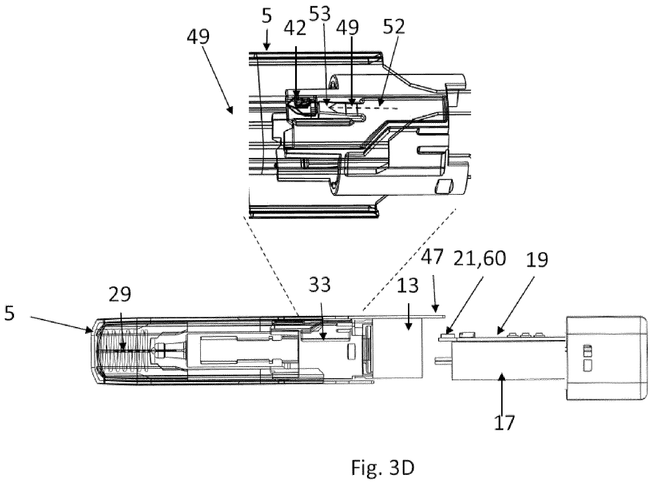

Fig. 3D illustrates side view of the medicament delivery device 1 after an injection event with the cover structure 5 in the extended position thereby protecting the needle 29. Here the electrical engine 13 has been deactivated.

Deactivation of the electrical engine 13 may be performed in various conceivable ways.

According to one example, the electrical engine 13 is configured to remain activated for a predetermined time duration. This time duration is sufficiently long to empty the medicament container using the electrical engine 13. For example, the electronic triggering device 19 is configured to deactivate the electrical engine 13 when the electronic non-contact sensor no longer detects the proximity of the sensing area 47. In other words, as long as the cover structure 5 is in the retracted position, the electrical engine 13 is active.

Further, when the cover structure moves from the retracted position to the extended position, the guiding pin 42 is received in the locking mechanism 49. The locking mechanism is arranged along a straight path indicated by dashed arrow 52 generally parallel with a longitudinal direction of the medicament delivery device 1. Once the guiding pin 42 is received in the locking mechanism 49, the cover structure 5 is locked in the retracted position. The guiding pin 41 is preferable locked in the locking mechanism 49 by a flexible stop member 53. The flexible stop 53 member is here exemplified as including a helical ramp surface which when being pushed in an axial direction flexes and allows the guiding pin 42 to reach a position proximal to the flexible stop member 53. Once in this position, the flexible stop 53 flexes back to its original shape, whereby the guiding pin 42 is not allowed to move in the distal direction, thereby locking the cover structure 5 in the extended position.

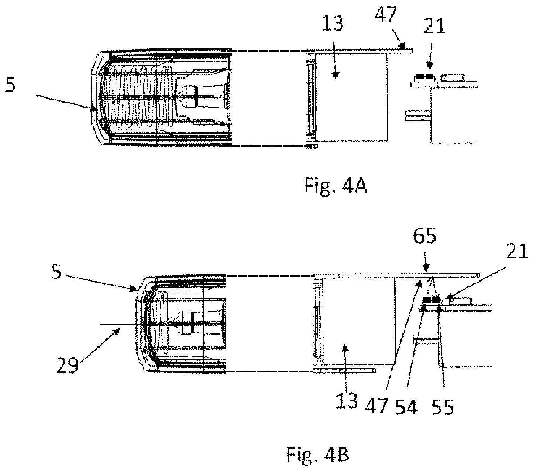

Figs. 4A-4B illustrate side views of a proximal portion comprising the cover structure 5 and a more distal portion where the electrical engine 13 and electronic triggering device 19 is located. Parts between these portions are omitted for clarity in figs 4A-4B.

The electronic triggering device 19 comprises an electronic non-contact sensor 21 comprising an emitter 54 configured to transmit a signal and a detector 55 configured to detect a reflected signal from the sensing area 47. whereby the triggering signal is provided in response to detecting the reflected signal.

In fig. 4A, the cover structure 5 is in the extended position whereby sensing area 47 is not detectable by the electronic non-contact sensor 21. With this type of sensor configured to emit a signal and detect a reflected signal, when the sensing area 47 is not located over the sensor, the emitted signal does not reach the sensing area 47, whereby no reflected signal from the sensing area is detected. The electronic non-contact sensor 21 is arranged spaced apart from the sensing area 47 such that the cover structure 5 can slide freely over the electronic non-contact sensor.

Now turning to fig. 4B, once the sensing area 47 is moved closer to the electronic non-contact sensor 21, here being directly above the electronic non-contact sensor 21, the emitted signal is reflected off the sensing area 47 and can be detected by the detector 55.

Various types of electronic non-contact sensors 21 relying on detecting a reflection are conceivable.

In one example, the electronic non-contact sensor 21 is an optical sensor, the emitter 53 being configured to emit an optical signal and the receiver 55 being configured to detect a reflected optical signal. In such case, the sensing area 47 may advantageously comprise an optically reflective surface. In one example, the optical sensor is an infrared sensor.

In a further example, the electronic non-contact sensor 21 is an ultrasound sensor, the emitter 53 being configured to emit an ultrasound signal and the receiver 55 being configured to detect a reflected ultrasound signal.

In a further example, the electronic non-contact sensor 21 is configured to sense the movable trigger by detecting an appearance of the sensing area 47. For example, if the electronic non-contact sensor 21 is a camera device, the electronic triggering device 19 may analyse images capture by the camera device, and once specific features such as marks present in the sensing area is recognized, it can be concluded that the sensing area is detected and the electrical engine can be activated.

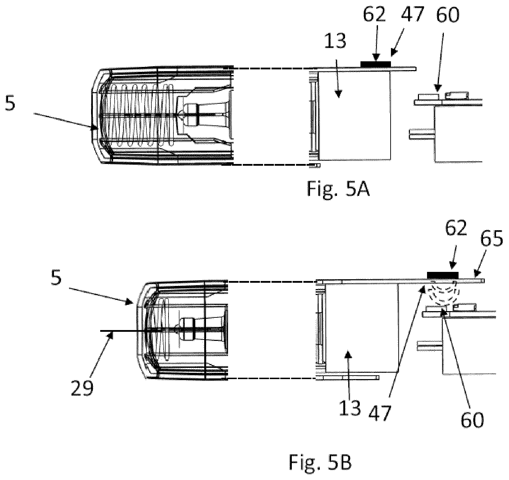

Figs. 5A-B illustrates similar side views as in figs. 4A-B without the housing. In this embodiment, electronic non-contact sensor is a magnetic field sensor 60 configured to detect a magnetic element 62 in the sensing area 47. The electronic triggering device is configured to provide a triggering signal when the detected magnetic field strength is above a detection threshold. The detection threshold may be calibrated so that the triggering signal is provided when the cover structure 5 is in the retracted position.

In fig. 5A, the cover structure 5 is in the extended position whereby magnetic field strength from magnetic element 62 at the magnetic field sensor 60 is below the detection threshold. In other words, the magnetic element 62 is too far from the magnetic field sensor 60 to cause actuation of the electrical engine 13.

In fig. 5B, the sensing area 47 including the magnetic element 62 is moved closer to the magnetic field sensor 60 due to the motion of the cover structure 5 being in the retracted position. Here the magnetic element 62 is directly above the magnetic field sensor 60. The magnetic field strength detected by the sensor 60 exceeds the detection threshold when the cover structure 5 is in this the retracted position whereby the electrical engine 13 is activated by the electronic triggering device 19.

In one example, the magnetic field sensor is a Hall effect sensor.

The motions of the sensing area 47 causes a variation in detection signal detected by the magnetic field sensor 60. A magnitude of this variation may be used for determining whether the movable trigger has been detected. Thus, if the variation exceeds a detection threshold, the movable trigger is determined to have been detected, whereby the electrical engine 13 is actuated.

In the examples shown in figs. 4A-B and figs. 5A-b, the cover structure 5 comprises the movable trigger in the form of an extension 65 comprising a sensing area 47. The extension 65 reaches in a distal direction of the cover structure 5 and is detectable by the electronic non-contact sensor. The electronic triggering device 19 is configured to sense that the cover structure is in the retracted position, and in response provide the triggering signal to activate the electrical engine 13.

In other possible implementations, the subassembly may comprise a push button configured to cause motion of the movable trigger. In other words, the subassembly may comprise a separate push button that is pressed by the user once the cover structure 5 is in the retracted position, whereby the movable trigger is sensed by the electronic non-contact sensor 21, 60.

Turning again to fig. 2, there is further provided a subassembly module 100 for controlling expulsion of medicament from a medicament container 7. The subassembly module 100 comprising a frame structure 15 arrangeable in a housing 11 having a proximal end 3c and a distal end 3b.

The subassembly module 100 comprises the electrical engine 13 configured to, upon activation, cause expulsion of the medicament out from the medicament container 7.

Further, an electronic triggering device 19 is configured to activate the electrical engine 13, and the power source 17 is configured to power the electrical engine 13 and the electronic triggering device 19.

The frame 15 is configured to accommodate the electrical engine 13, the power source 17, and the electronic triggering device 19. The frame 15 is located in the distal first housing part 9. The frame 15 is thus distal to the cover structure 5 and the medicament container holder 38. Further, the frame is located such that the electrical engine 13 can cause a plunger to move in the medicament container 7 by the force from the electrical engine 13 when the electrical engine 13 is activated. Thus, the frame is advantageously adjacent to the medicament container 7, when in use.

The electronic triggering device 19 comprises an electronic non-contact sensor 21, 60 configured to sense the movable trigger 6 comprising a sensing area 47. In response to sensing the sensing area the electronic triggering device 19 is configured to provide a triggering signal to activate the electrical engine 19.

Accordingly, the frame 15 comprises an inner space for accommodating the electrical engine 13, the power source 17, and the electronic triggering device 19.

The electronic triggering device 19 may comprising processing circuitry configured to receive a sensing signal from the electronic non-contact sensor 21 indicative of the sensing area 47 being sensed, and in response to provide an activation signal to the electrical engine 13. The electronic triggering device further includes a printed circuit board, PCB 68, for carrying the processing circuitry and the electronic non-contact sensor 21, 60.

Such processing circuitry may comprise a logic circuit or control unit including a microprocessor, microcontroller, programmable digital signal processor or another programmable device. The processing circuitry may also, or instead, each include an application specific integrated circuit, a programmable gate array or programmable array logic, a programmable logic device, or a digital signal processor. Where the processing circuitry includes a programmable device such as the microprocessor, microcontroller or programmable digital signal processor mentioned above, the processor may further include computer executable code that controls operation of the programmable device.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A subassembly (2) of a medicament delivery device (1) for expelling medicament from a medicament container (7) of the medicament delivery device, the subassembly comprising:
a housing (3) having a proximal end (3a) and a distal end (3b);
an electrical engine (13) configured to, upon activation cause expulsion of the medicament out from the medicament container
a cover structure (5) configured to surround a medicament delivery member (29) at the proximal end of the housing (3), the cover structure (5) is movable in a longitudinal direction of the housing (3) between a retracted position in which the medicament delivery member (29) is exposed at the proximal end of the cover structure (5), and an extended position with the medicament delivery member (29) covered by the cover structure (5);
an electronic triggering device (19) configured to activate the electrical engine (13), the electrical engine (13) and the electronic triggering device (19) being accommodated in the housing (3);
the electronic triggering device (19) comprises an electronic non-contact sensor (21, 60) configured to sense a movable trigger (6) comprising a sensing area (47) wherein,
the cover structure (5) comprises the sensing area (47), wherein the electronic triggering device (9) is configured to sense that the cover structure (5) is in the retracted position, and wherein
in response to sensing that the cover structure (5) is in the retracted position, the electronic triggering device (19) is configured to provide a triggering signal to activate the electrical engine (13).

2. The subassembly according to claim 1, the electronic non-contact sensor comprising an emitter (54) configured to transmit a signal and a detector (55) configured to detect a reflected signal from the sensing area, whereby the triggering signal is provided in response to detecting the reflected signal.

3. The subassembly according to claim 2, wherein the electronic non-contact sensor is an optical sensor or an ultrasound sensor.

4. The subassembly according to claim 1, the electronic non-contact sensor being a magnetic field sensor (60) configured to detect a magnetic element in the sensing area, wherein the electronic triggering device is configured to provide a triggering signal when the detected magnetic field strength is above a detection threshold.

5. The subassembly according to any one of claims 1-4 wherein the electronic non-contact sensor is configured to sense the movable trigger by detecting a variation in detection signal caused by a motion of the sensing area.

6. The subassembly according to any one of the preceding claims, wherein the electrical engine is configured to generate propellant gas upon being activated.

7. The subassembly according to claim 6, wherein the electrical engine comprises liquid substance and configured to generate propellant gas from the liquid substance upon being activated.

8. The subassembly according to any one of claims 6 and 7, wherein the propellant gas is configured to flow into the medicament container and propel a plunger of the medicament container in the proximal direction of the medicament container.

9. The subassembly according to any of the claims 7-8, wherein the subassembly comprises a plunger for expelling a contained medicament in the medicament container; wherein the electrical engine is connected to the plunger, so that the propellant gas is configured to propel the plunger in the proximal direction of the medicament container upon the electrical engine is activated.

10. The subassembly according to claim 8, wherein the subassembly comprises a medicament container holder for holding the medicament container; wherein the medicament container holder is gas-tightly sealed to the electrical engine.

11. The subassembly according to claim 10, wherein the subassembly comprises a resilient ring positioned between the medicament container and holder and the electrical engine; and wherein the medicament container holder is connected to the electrical engine through a screw thread engagement.

12. The subassembly according to any of the claims 6 and 7, wherein the propellant gas is configured to flow into a tubular body of the plunger.

13. The subassembly according any of the claims 6 and 7, wherein the propellant gas is configured to flow into a sleeve connected to either a tubular plunger or a solid core plunger rod.

## Patentansprüche

1. Unteranordnung (2) einer Arzneimittelabgabevorrichtung (1) zum Ausstoßen von Arzneimittel aus einem Arzneimittelbehälter (7) der Arzneimittelabgabevorrichtung, die Unteranordnung umfassend:
ein Gehäuse (3), das ein proximales Ende (3a) und ein distales Ende (3b) aufweist;
einen Elektromotor (13), der konfiguriert ist, um, bei Aktivierung, einen Ausstoß des Arzneimittels aus dem Arzneimittelbehälter zu veranlassen
eine Abdeckstruktur (5), die konfiguriert ist, um ein Arzneimittelabgabeteil (29) an dem proximalen Ende des Gehäuses (3) zu umgeben, wobei die Abdeckstruktur (5) in eine Längsrichtung des Gehäuses (3) zwischen einer eingefahrenen Position, in der das Arzneimittelabgabeteil (29) an dem proximalen Ende der Abdeckstruktur (5) freiliegt, und einer ausgefahrenen Position, in der das Arzneimittelabgabeteil (29) durch die Abdeckstruktur (5) abgedeckt ist, bewegbar ist;
eine elektronische Auslösevorrichtung (19), die konfiguriert ist, um den Elektromotor (13) zu aktivieren, wobei der Elektromotor (13) und die elektronische Auslösevorrichtung (19) in dem Gehäuse (3) aufgenommen sind;
wobei die elektronische Auslösevorrichtung (19) einen elektronischen berührungslosen Sensor (21, 60), der konfiguriert ist, um einen bewegbaren Auslöser (6), umfassend einen Erfassungsbereich (47), zu erfassen, umfasst, wobei
die Abdeckstruktur (5) den Erfassungsbereich (47) umfasst, wobei die elektronische Auslösevorrichtung (9) konfiguriert ist, um zu erfassen, dass die Abdeckstruktur (5) in der eingefahrenen Position ist, und wobei
als Reaktion auf das Erfassen, dass die Abdeckstruktur (5) in der eingefahrenen Position ist, die elektronische Auslösevorrichtung (19) konfiguriert ist, um ein Auslösesignal bereitzustellen, um den Elektromotor (13) zu aktivieren.

2. Unteranordnung nach Anspruch 1, der elektronische berührungslose Sensor umfassend einen Emitter (54), der konfiguriert ist, um ein Signal zu übertragen, und einen Detektor (55), der konfiguriert ist, um ein reflektiertes Signal aus dem Erfassungsbereich zu detektieren, wobei das Auslösesignal als Reaktion auf das Detektieren des reflektierten Signals bereitgestellt wird.

3. Unteranordnung nach Anspruch 2, wobei der elektronische berührungslose Sensor ein optischer Sensor oder ein Ultraschallsensor ist.

4. Unteranordnung nach Anspruch 1, wobei der elektronische berührungslose Sensor ein Magnetfeldsensor (60) ist, der konfiguriert ist, um ein magnetisches Element in dem Erfassungsbereich zu detektieren, wobei die elektronische Auslösevorrichtung konfiguriert ist, um ein Auslösesignal bereitzustellen, wenn die detektierte Magnetfeldstärke über einer Detektionsschwelle liegt.

5. Unteranordnung nach einem der Ansprüche 1 bis 4, wobei der elektronische berührungslose Sensor konfiguriert ist, um den bewegbaren Auslöser durch Detektieren einer Variation in einem Detektionssignal, die durch eine Bewegung des Erfassungsbereichs veranlasst wird, zu erfassen.

6. Unteranordnung nach einem der vorstehenden Ansprüche, wobei der Elektromotor konfiguriert ist, um, wenn er aktiviert wird, Treibgas zu erzeugen.

7. Unteranordnung nach Anspruch 6, wobei der Elektromotor flüssige Substanz umfasst und konfiguriert ist, um, wenn er aktiviert wird, Treibgas aus der flüssigen Substanz zu erzeugen.

8. Unteranordnung nach einem der Ansprüche 6 und 7, wobei das Treibgas konfiguriert ist, um in den Arzneimittelbehälter zu strömen und einen Kolben des Arzneimittelbehälters in die proximale Richtung des Arzneimittelbehälters zu treiben.

9. Unteranordnung nach einem der Ansprüche 7 bis 8, wobei die Unteranordnung einen Kolben zum Ausstoßen eines enthaltenen Arzneimittels in dem Arzneimittelbehälter umfasst; wobei der Elektromotor mit dem Kolben verbunden ist, sodass das Treibgas konfiguriert ist, um den Kolben in die proximale Richtung des Arzneimittelbehälters zu treiben, wenn der Elektromotor aktiviert wird.

10. Unteranordnung nach Anspruch 8, wobei die Unteranordnung einen Arzneimittelbehälterhalter zum Halten des Arzneimittelbehälters umfasst; wobei der Arzneimittelbehälterhalter gegenüber dem Elektromotor gasdicht abgedichtet ist.

11. Unteranordnung nach Anspruch 10, wobei die Unteranordnung einen elastischen Ring, der zwischen dem Arzneimittelbehälter und Halter und dem Elektromotor positioniert ist, umfasst; und wobei der Arzneimittelbehälterhalter durch einen Schraubengewindeeingriff mit dem Elektromotor verbunden ist.

12. Unteranordnung nach einem der Ansprüche 6 und 7, wobei das Treibgas konfiguriert ist, um in einen rohrförmigen Körper des Kolbens zu strömen.

13. Unteranordnung nach einem der Ansprüche 6 und 7, wobei das Treibgas konfiguriert ist, um in eine Hülse, die entweder mit einem rohrförmigen Kolben oder einer Kolbenstange mit festen Kern verbunden ist, zu strömen.

## Revendications

1. Sous-ensemble (2) d'un dispositif d'administration de médicament (1) destiné à expulser un médicament à partir d'un récipient à médicament (7) du dispositif d'administration de médicament, le sous-ensemble comprenant :
un boîtier (3) présentant une extrémité proximale (3a) et une extrémité distale (3b) ;
un moteur électrique (13) configuré pour, lors de l'activation, provoquer une expulsion du médicament hors du récipient à médicament
une structure de recouvrement (5) conçue pour entourer un élément d'administration de médicament (29) au niveau de l'extrémité proximale du boîtier (3), la structure de recouvrement (5) est mobile dans une direction longitudinale du boîtier (3) entre une position rétractée, dans laquelle l'élément d'administration de médicament (29) est apparent au niveau de l'extrémité proximale de la structure de recouvrement (5), et une position étendue, l'élément d'administration de médicament (29) étant recouvert par la structure de recouvrement (5) ;
un dispositif de déclenchement électronique (19) configuré pour activer le moteur électrique (13), le moteur électrique (13) et le dispositif de déclenchement électronique (19) étant logés dans le boîtier (3) ;
le dispositif de déclenchement électronique (19) comprend un capteur électronique sans contact (21, 60) configuré pour détecter un déclencheur mobile (6) comprenant une zone de détection (47) dans lequel,
la structure de recouvrement (5) comprend la zone de détection (47), dans lequel le dispositif de déclenchement électronique (9) est configuré pour détecter que la structure de recouvrement (5) est dans la position rétractée, et dans lequel,
en réponse à la détection du fait que la structure de recouvrement (5) est dans la position rétractée, le dispositif de déclenchement électronique (19) est configuré pour fournir un signal de déclenchement afin d'activer le moteur électrique (13).

2. Sous-ensemble selon la revendication 1, le capteur électronique sans contact comprenant un émetteur (54) configuré pour transmettre un signal et un détecteur (55) configuré pour détecter un signal réfléchi provenant de la zone de détection, moyennant quoi le signal de déclenchement est fourni en réponse à la détection du signal réfléchi.

3. Sous-ensemble selon la revendication 2, dans lequel le capteur électronique sans contact est un capteur optique ou un capteur à ultrasons.

4. Sous-ensemble selon la revendication 1, le capteur électronique sans contact étant un capteur de champ magnétique (60) configuré pour détecter un élément magnétique dans la zone de détection, dans lequel le dispositif de déclenchement électronique est configuré pour fournir un signal de déclenchement lorsque l'intensité de champ magnétique détectée est supérieure à un seuil de détection.

5. Sous-ensemble selon l'une quelconque des revendications 1 à 4, dans lequel le capteur électronique sans contact est configuré pour détecter le déclencheur mobile par détection d'une variation du signal de détection provoquée par un mouvement de la zone de détection.

6. Sous-ensemble selon l'une quelconque des revendications précédentes, dans lequel le moteur électrique est configuré pour générer du gaz propulseur lors de son activation.

7. Sous-ensemble selon la revendication 6, dans lequel le moteur électrique comprend une substance liquide et est configuré pour générer du gaz propulseur à partir de la substance liquide lors de son activation.

8. Sous-ensemble selon l'une quelconque des revendications 6 et 7, dans lequel le gaz propulseur est conçu pour s'écouler dans le récipient à médicament et propulser un piston du récipient à médicament dans le sens proximal du récipient à médicament.

9. Sous-ensemble selon l'une quelconque des revendications 7 et 8, dans lequel le sous-ensemble comprend un piston destiné à expulser un médicament contenu dans le récipient à médicament ; dans lequel le moteur électrique est connecté au piston, de telle sorte que le gaz propulseur est conçu pour propulser le piston dans le sens proximal du récipient à médicament lors de l'activation du moteur électrique.

10. Sous-ensemble selon la revendication 8, dans lequel le sous-ensemble comprend un support de récipient à médicament destiné à supporter le récipient à médicament ; dans lequel le support de récipient à médicament est scellé de manière hermétique au gaz sur le moteur électrique.

11. Sous-ensemble selon la revendication 10, dans lequel le sous-ensemble comprend un anneau élastique positionné entre le récipient à médicament et le support d'une part et le moteur électrique d'autre part ; et dans lequel le support de récipient à médicament est relié au moteur électrique par le biais d'une mise en prise par filetage.

12. Sous-ensemble selon l'une quelconque des revendications 6 et 7, dans lequel le gaz propulseur est conçu pour s'écouler dans un corps tubulaire du piston.

13. Sous-ensemble selon l'une quelconque des revendications 6 et 7, dans lequel le gaz propulseur est conçu pour s'écouler dans un manchon relié soit à un piston tubulaire, soit à une tige de piston à âme pleine.
